# EUROPEAN PATENT SPECIFICATION

(11) **EP 3 659 673 B1**
(45) Date of publication and mention of the grant of the patent: **20.12.2023**
(21) Application number: 18208832.8
(22) Date of filing: 28.11.2018
(51) Int. Cl.: A61Q 5/06, A61K 8/81, A61K 8/25, A61K 8/19, A61K 8/73, A61K 8/34, A61K 8/04, A61K 8/31, A61K 8/33, A61K 8/02

(54) **AEROSOL COMPOSITION FOR KERATIN FIBERS**
AEROSOLZUSAMMENSETZUNG FÜR KERATINFASERN
COMPOSITION AÉROSOL POUR FIBRES DE KÉRATINE

(43) Date of publication of application: 03.06.2020
(73) Proprietor: Kao Corporation, Chuo-ku Tokyo 103-8210 (JP)
(72) Inventor: Sulzbach, Melina, 64297 Darmstadt (DE); Schmid, Sabine, 64297 Darmstadt (DE)
(74) Representative: Hoffmann Eitle

(56) References cited:
- US-A1- 2009 041 683
- US-A1- 2015 104 397
- US-A1- 2016 038 398
- US-A1- 2016 075 501
- US-A1- 2018 000 699

## Description

### Field of the invention

The present invention is in the field of aerosol compositions for cosmetic application, which deliver texture benefits for keratin fibers.

### Background of the invention

Customers with fine hair structures have common daily challenges to create a hair style corresponding to their personal taste. Typically the hair of the aforementioned target group suffers from a lack of volume which is even worse after washing the hair or after getting it wet. Consequently, cosmetic industry offers products for increasing volume while simultaneously allowing to create different fashionable hair styles.

However, many products have disadvantages such as insufficient volumizing effect, slow drying after application, and low durability of the styling effect.

Usually, compositions enhancing volume of keratin fibers are water-based as disclosed in Mintel 1072700 and US 2002034486. Water-free compositions are also known from WO97/30681, Mintel 5584809, and Mintel 3271413.

However, none of the prior art documents disclose compositions which are quick-drying, enhance volume and body of keratin fibers, and deliver a treatment being resistant to average environmental stress factors such as temperature and humidity.

The inventors of the present invention have unexpectedly found that an aerosol composition comprising different film-forming polymers, organic and/or inorganic particulates, and certain organic solvents overcome the disadvantages of the prior art.

US 2016/075501 A1 and US 2015/104397 A1 describe aerosol compositions for the hair, comprising a fixing polymer, particles and a propellant.

US 2016/038398 A1 concerns an aerosol hair styling composition comprising an aqueous, aqueous/alcoholic or alcoholic medium, starch and a propellant.

US 2009/041683 A1 relates to a hair stylinc composition comprising at least one film forming polymer and at least one arylated silicone.

US 2018/0000699A1 relates to hairspray compositions comprising a liquid carrier, hollow, fluid-filled microspheres, a polymer fixative and a propellant.

### Summary of the Invention

Therefore, the first object of the present invention is an aerosol composition comprising:
- One or more non-silicone non-ionic film forming polymer(s),
- One or more anionic film forming polymer(s), and/or its/their salt(s), and/or their mixtures,
- One or more organic and/or inorganic particulate being insoluble in organic solvents having a boiling point in the range of 50°C to 120°C under atmospheric conditions,
- One or more organic solvent(s) having a boiling point in the range of 50°C to 120°C under atmospheric conditions,
- One or more propellant(s),
wherein the composition comprises less than 5% by weight of water, preferably less than 1% by weight of water, calculated to the total of the composition, more preferably it is anhydrous.

The second object of the present invention is a method for styling and/or increasing volume and/or body of keratin fibers, preferably human keratin fibers, more preferably human hair, characterized in that it comprises the steps of:
a) optionally shampooing and drying the keratin fibers,
b) applying to dry keratin fibers a composition as defined above,
c) optionally styling the keratin fibers using a comb or brush.

The third object of the present invention is a vaporizer comprising the composition as defined above.

The composition of the present invention can be part of a kit-of-parts comprising in a separately packed container the composition as defined above and a hair styling device, preferably a comb or a brush.

### Detailed description of the invention

Film forming polymers within the meaning of the present invention are polymers which are able to form a film upon spraying a polymer solution and/or dispersion on a solid substrate and allowing the solvent to evaporate. During solvent evaporation the polymer chains become entangled which eventually leaves a film covering the solid substrate.

Volume and body of keratin fibers are used interchangeably in the present application because both properties describe volume of keratin fibers. However, in general volume is an optical property of keratin fibers, i.e., it can be determined by optical instruments or the naked human eye.

Body is a haptic property of keratin fibers, i.e., it can be determined by sensors touching the keratin fiber surface or by touching/feeling with the human hand.

### Non-ionic film-forming polymers

Suitable non-ionic film forming polymers which are free of silicone units preferably comprise monomer units selected from vinylpyrrolidone, vinylacetate, vinylcaprolactam.

Exemplified suitable non-ionic polymers are vinylpyrrolidone polymers of either homopolymers or copolymers with, particularly, vinylacetate, commonly known as VP/VA copolymers. Those homopolymers are known with the trade name "Luviskol" as Luviskol K 30, K 60 or K 90, whereas the VP/VA copolymers are known as Luviskol VA 55, VA 64, or Plus from BASF AG.

Natural non-ionic polymers are as well suitable for the composition of the present invention. Those are such as cellulose, guar gum, and neutralised shellac, and/or their derivatives.

The preferred non-silicone non-ionic polymers are vinylacetate / vinylpyrrolidone copolymers from the viewpoint of achieving sufficient texture and long-lasting hold of keratin fibers.

The total concentration of one or more non-silicone non-ionic film forming polymer(s) preferably is 0.1% by weight or more, more preferably of 0.2% by weight or more, further more preferably of 0.5% by weight or more, calculated to the total of the composition, from the viewpoint of achieving sufficient texture and long-lasting hold of keratin fibers.

The total concentration of one or more non-silicone non-ionic film forming polymer(s) polymer(s) preferably is 10% by weight or less, more preferably 8% by weight or less, further more preferably 5% by weight or less, calculated to the total of the composition, from the viewpoint of the sprayability of the composition.

For attaining the above-mentioned effects, the composition comprises one or more non-silicone non-ionic film forming polymer(s) at a total concentration in the range of 0.1% to 10% by weight, preferably in the range of 0.2% to 8% by weight, more preferably in the range of 0.5% to 5% by weight, calculated to the total of the composition.

### Anionic film-forming polymers

Suitable anionic polymers include in particular vinyl acetate/crotonic acid or vinyl acetate/vinylneodecanoate/crotonic acid copolymers of the type "Resyn^{®}"; sodium acrylate/vinyl alcohol copolymers of the type "Hydagen^{®} F", sodium polystyrene sulfonate, e.g. "Flexan^{®} 130"; ethylacrylate/acrylic acid/N-tert-butyl acrylamide copolymers of the type "Ultrahold^{®}"; vinyl pyrroliydone/vinylacetate/itaconic acid copolymers, acrylic acid/acrylamide copolymers or the sodium salts thereof of the type "RetenO".

Non-acrylate copolymers are suitable as well, for example vinylcaprolactam/PVP/dimethylaminoethylmethacrylate copolymer available under Advantage LC-E from ISP Corp. and VA/crotonates copolymer available under trade name Luviset CA 66 from BASF Corp. Equally suitable is isobutylene/ethylmaleimide/hydroxyethyl maleimide copolymer marketed under the trade name Aquaflex FX-64 by Ashland Corp.

Further suitable anionic polymers are vinyl alkyl ether, in particular methyl vinyl ether/maleic acid copolymers, obtained by hydrolysis of vinyl ether/maleic anhydride copolymers, distributed under the trade name "Gantrez^{®} AN or ES". These polymers may also be partly esterified, as for example, "Gantrez^{®} ES 225" or "ES 435", the ethyl ester of an ethyl vinyl ether/maleic acid copolymer, or the butyl or isobutyl ester thereof.

It is preferred from the viewpoint of film-forming properties that the composition comprises one or more anionic film forming polymer and/or its/their salt(s), and/or its/their mixtures, preferably having acrylate and/or alkyl acrylate and/or acrylamide and/or alkyl acrylamide monomers. Suitable polymers are summarized under the CTFA umbrella name acrylates copolymer. Other suitable acrylate copolymers are available under the CTFA name acrylates/t-butylacrylamide copolymer available under trade name Ultrahold Strong and Ultrahold Power by BASF Corp., PEG/PPG 25/25 dimethicone / acrylate copolymer available under trade name Luviflex Silk from BASF Corp.

It is to be noted that suitable anionic polymers are used either alone or in mixture with each other.

A particularly preferred anionic polymer for the composition of the present invention is acrylates/t-butylacrylamide copolymer, and/or its salts and/or mixtures, from the viewpoint of achieving a high texture, high keratin fiber body and volume.

The total concentration of anionic film forming polymers, and/or its/their salts, in the composition of the present invention preferably is 0.1% by weight or more, more preferably 0.2% by weight or more, calculated to the total of the composition, from the viewpoint of achieving a high texture, high keratin fiber body and volume.

The total concentration of anionic film forming polymers, and/or its/their salts, in the composition of the present invention preferably is 10% by weight or less, more preferably 8% by weight or less, further more preferably 5% by weight or less, calculated to the total of the composition, from the viewpoint of the sprayability of the composition.

For attaining the above-mentioned effect, the total concentration of anionic film forming polymers, and/or its/their salts, is in the range of 0.1% to 10% by weight, preferably in the range of 0.2% to 8% by weight, more preferably in the range of 0.2% to 5% by weight, calculated to the total of the composition.

### Organic / inorganic particulates

For the purpose of the present invention, namely from the viewpoint of providing high texture on keratin fibers, suitable organic and/or inorganic particulates are at least partially, preferably completely insoluble in organic solvents and propellants.

Preferably, the composition of the present invention comprises one or more organic and/or inorganic particulate with an average particle size in the range of 1 µm to 250 µm, more preferably in the range of 10 µm to 100 µm, from the viewpoint of achieving high keratin fiber roughness and long durability of the styling result. In this connection, the average particle size is understood as a volume average value, which may, e.g., be determined by the laser scattering method.

Suitably, the one or more organic and/or inorganic particulate does not confer color to hair, judged by the naked human eye under daylight conditions while spraying 0.5 g of the composition onto 2 g of keratin fibers.

Suitable inorganic particulates include amorphous silica, calcium carbonate, calcium sulfate, diatomaceous earth, zeolithe, and perlite.

Suitable organic particulates include vegetable starch and/or its synthetically modified derivatives. Examples for vegetable starch are rice starch, potato starch, tapioca starch, and wheat starch. Examples for synthetically modified derivatives of starches are aluminium starch octenylsuccinate, hydroxypropyl starch phosphate, and/or their cosmetically acceptable salts.

A particularly preferred inorganic particulate for the composition of the present invention from the viewpoint of sprayability of the composition and achieving a high texture effect on keratin fibers is amorphous silica and/or calcium carbonate.

The total concentration of one or more organic and/or inorganic particulate usually may be 0.25% by weight or more, preferably 0.5% by weight or more, more preferably 1% by weight or more, calculated to the total of the composition, from the viewpoint of achieving a high texture effect on keratin fibers.

The total concentration of one or more organic and/or inorganic particulate usually may be 10% by weight or less, preferably 8% by weight or less, more preferably 5% by weight or less, calculated to the total of the composition, from the viewpoint of sprayability and viscosity of the composition.

For attaining the above-mentioned effects, the total concentration of one or more organic and/or inorganic particulate usually may be in the range of 0.25% to 10% by weight, preferably in the range of 0.5% to 8% by weight, more preferably in the range of 1% to 5% by weight, calculated to the total of the composition.

It is further preferred from the viewpoint of achieving a homogenous film on the keratin fibers that the weight ratio of non-silicone non-ionic film forming polymer to anionic film forming polymer(s) is in the range of 0.2 to 5.

It is further preferred from the viewpoint of achieving good adhesion of inorganic and/or organic particulates to keratin fibers that the weight ratio of total concentration of non-ionic film forming polymer and anionic film forming polymer(s) to total concentration of inorganic and/or organic particulates is in the range of 0.5 to 10.

### Organic solvents

The purpose of the organic solvent for the composition of the present invention is to dissolve the polymers and to disperse the organic and/or inorganic particulates.

Suitable organic solvents include ethanol, n-propanol, iso-propanol, and/or their mixtures.

A particularly preferred organic solvent is ethanol from the viewpoint of low boiling point, quick drying properties, and having an acceptable smell upon evaporation.

The amount of organic solvent in the composition of the present invention should be sufficient to completely dissolve the film forming polymers while maintaining an acceptable composition viscosity for spraying. Thus, from the viewpoint of sprayability of the composition, the viscosity of the composition of the present invention preferably is in the range of 1 mPas to 20,000 mPas, determined with a falling ball viscometer at 25°C. A device and method for measuring viscosity of aerosol compositions is disclosed in EP2746745. In principle, the liquid composition is filled into the viscometer and the propellant is added into the air-tight viscometer to simulate the conditions of an aerosol can.

The total concentration of organic solvent in the composition is preferably in the range of 5% to 50% by weight, more preferably in the range of 10% to 45% by weight, calculated to the total of the composition from the view point of dissolving the polymers, dispersing the organic and/or inorganic particulates and sprayability of the composition.

### Propellants

The composition of the present invention is an aerosol composition and comprises one or more propellant(s). In principle, all known cosmetically acceptable propellants are suitable for the present invention. Suitably the propellant is selected from carbon dioxide, dimethylether, n-butane, iso-butane, liquefied petroleum gas, hydrofluorcarbon gases, and/or their mixtures, from the viewpoint of quick drying of the composition.

The composition comprises propellants at a total concentration of at least 40% by weight, preferably 55% to 90% by weight, more preferably 60% to 85% by weight, calculated to the total of the composition from the viewpoint of achieving small droplet size during spraying and allowing for quick drying of the composition.

Depending on the type of aerosol can and type of propellant used, the pressure inside the aerosol can is usually kept below 12 bar, preferably it is around 8.5 bar, further preferably it is below 5 bar from the viewpoint of safety.

### Optional Ingredients

The composition of the present invention may optionally comprise conditioning components such as silicones, cationic polymers and/or cationic surfactants, as well as further additives such as direct dyes and/or UV filters.

### Silicone polymers

The composition of the present invention may comprise polymers having silicone units such as dimethylpolysiloxanes, and modified silicones (for example, amino-modified silicones, fluorine-modified silicones, alcohol-modified silicones, polyether-modified silicones, epoxy-modified silicones, or alkyl-modified silicones), and dimethylpolysiloxane, polyether-modified silicones and amino-modified silicones are preferred. Amino-modified silicones are commonly known under their CTFA name amodimethicone.

The dimethylpolysiloxane may be any cyclic or non-cyclic dimethylsiloxane polymer, and examples thereof include SH200 series, BY22-019, BY22-020, BY11-026, B22-029, BY22-034, BY22-050A, BY22-055, BY22-060, BY22-083, FZ-4188 (all by Dow Corning Toray Co., Ltd.), KF-9008, KM-900 series, MK-15H, and MK-88 (all by Shin-Etsu Chemical Co., Ltd.).

The polyether-modified silicone may be any silicone having a polyoxyalkylene group, and the group constituting the polyoxyalkylene group may be an oxyethylene group or an oxypropylene group. More specific examples include KF-6015, KF-945A, KF-6005, KF-6009, KF-6013, KF-6019, KF-6029, KF-6017, KF-6043, KF-353A, KF-354A, KF-355A (all by Shin-Etsu Chemical Corp.), FZ-2404, SS-2805, FZ-2411, FZ-2412, SH3771M, SH3772M, SH3773M, SH3775M, SH3749, SS-280X series, BY22-008 M, BY11-030, and BY25-337 (all by Dow Corning Toray Corp.).

Specific examples of suitable commercially available amodimethicone oils such as SF8452C, SS-3551 (all by Dow Corning Toray Co., Ltd.), KF-8004, KF-867S, and KF-8015 (all by Shin-Etsu Chemical Corp.).

The total concentration of silicone polymers in the composition of the present invention may be in the range of 0.01% to 1% by weight, calculated to the total of the composition, from the viewpoint of improving the film-forming properties of the composition.

### Cationic film forming polymers

The composition of the present invention preferably comprises one or more cationic film forming polymers having units of silicone(s) and/or is/are free of silicone units.

It is preferred from the viewpoint of achieving a high cationic charge density and adhesion to keratin fibers that the cationic film polymers comprise monomer units having a quaternized ammonium group.

Suitable cationic polymers being free of silicone units include Polyquaternium 6, Polyquaternium 7, Polyquaternium 10, Polyquaternium 11, Polyquaternium 16, Polyquaternium 22, Polyquaternium 24, Polyquaternium 28, Polyquaternium 30, Polyquaternium 36, Polyquaternium 37, Polyquaternium 46, , Polyquaternium 67, and Polyquaternium 72.

Suitable cationic polymers having silicone units include organopolysiloxane graft polymers, for example the polymers known under their CTFA name Polysilicone-1, Polysilicone-9, Polysilicone-14, Polysilicone-16, Polysilicone-18, Polysilicone-19, Polysilicone-24, Polysilicone-25, Polysilicone-26, Polysilicone-27, and Polysilicone-28.

Particularly preferred cationic film-forming polymers in the composition of the present invention are Polysilicone-9 and/or Polysilicone-28 from the viewpoint of achieving a high surface roughness and achieving high volume and body on keratin fibers.

The total concentration of one or more cationic film forming polymer(s) preferably is 0.001% by weight or more, more preferably of 0.01% by weight or more, further more preferably of 0.1% by weight or more, calculated to the total of the composition, from the viewpoint of achieving sufficient texture and long-lasting hold of keratin fibers.

The total concentration cationic film forming polymer(s) preferably is 5% by weight or less, more preferably 3% by weight or less, further more preferably 2% by weight or less, calculated to the total of the composition, from the viewpoint of the sprayability of the composition.

For attaining the above-mentioned effects, the composition comprises cationic film forming polymer(s) at a total concentration in the range of 0.001% to 5% by weight, preferably in the range of 0.01% to 3% by weight, more preferably in the range of 0.1% to 2% by weight, calculated to the total of the composition.

### Cationic surfactants

The composition of the present invention may also comprise one or more cationic surfactants. Suitable ones are known under their CTFA name Quaternium, for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-81, Quaternium-82, Quaternium-83 and Quaternium-84.

The concentration of cationic surfactants usually may be in the range of 0.001% to 5% by weight, preferably in the range of 0.01% to 3% by weight, more preferably in the range of 0.1 % to 2% by weight, calculated to the total of the composition.

### Direct Dyes

The composition of the present invention may additionally comprise one or more hair direct dye(s) from the viewpoint of conferring color to the composition, but not to the keratin fibers. Preferably they are selected from cationic and/or anionic and/or non-ionic hair direct dyes, and/or their mixtures.

The total concentration of hair direct dyes in the composition of the present invention is in the range of 0.01% to 1% by weight, calculated to the total of the composition.

### UV Filters

The composition of the present invention may further comprise one or more UV filter(s) which is soluble in the organic solvent selected for the present composition. UV filters or mixtures thereof protect the keratin fibers from damaging effects of sun light. The UV-filters are preferably selected from the following compounds: 4-Aminobenzoic acid and/or esters and/or salts thereof, 2-phenyl benzimidazole-5-sulfonic acid and/or alkali and/or amine salts thereof, 4-dimethyl aminobenzoic acid and/or esters and/or salts thereof, cinnamic acid and/or esters and/or salts thereof, 4-methoxycinnamic acid and/or esters and/or salts thereof, salicylic acid and/or esters and/or salts thereof, 2,4-dihydroxybenzophenone, 2,2',4,4'-tetrahydroxybenzophenone, 2-hydroxy-4-methoxybenzophenone and its 5-sulfonic acid or the sodium salt thereof, 2,2'-dihydroxy-4,4'-dimethoxybenzophenone, 2-hydroxy-5-chlorobenzophenone, 2,2'-dihydroxy-4-methoxybenzophenone, 2,2'-dihydroxy-4,4'-dimethoxy-5,5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and/or salts thereof and/or 3-(4'-methyl benzylidene)-DL-campher.

The preferred amount of the UV filter in the composition of the present invention is in the range from 0.01 % to 2.5%, more preferably in the range from 0.05 % to 1 % by weight, calculated to the total of the composition.

The following examples are to illustrate the invention.

### EXAMPLES

### Example 1

The following compositions were prepared by conventional formulation and mixing techniques:

| **Ingredients** | | **Inventive comp. 1** | **Inventive comp. 2** | **Inventive comp. 3** | **Inventive comp. 4** | **Inventive comp. 5** | **Comparative comp. 1** |
|---|---|---|---|---|---|---|---|
| | | **% by weight** | | | | | |
| **Non-ionic film forming polymer** | **VP/VA copolymer*** | 1.0 | 1.0 | 1.0 | 1.0 | 1.0 | 2.5 |
| **Anionic film forming polymer** | **Acrylates/ t-Butylacrylamide copolymer**** | 1.5 | 1.5 | 1.5 | 1.5 | 1.5 | - |
| **Particulates** | **Amorphous silica***** | 1.5 | - | - | - | - | - |
| | **Calcium carbonate****** | - | 1.5 | - | - | - | 1.5 |
| | **Zeolite******* | - | - | 1.5 | - | - | - |
| | **Rice starch******** | - | - | - | 1.5 | - | - |
| | **Perlite********* | - | - | - | - | 1.5 | - |
| **Organic solvent** | **Ethanol** | 15.0 | 15.0 | 15.0 | 15.0 | 15.0 | 15.0 |
| **Propellant** | **Dimethylether** | Ad 100.0 | | | | | |

| | | | | | | | |
|---|---|---|---|---|---|---|---|
| * Luviskol VA 64 ** Ultrahold Power by BASF Corp. *** Goldmann Silica 800, average particle sizes between 10-20 µm **** Food grade, average particles sizes between 10-65 µm ***** Natural zeolite, average particle sizes between 10-100 µm ****** Natural rice starch, food grade, average particle sizes -15 µm ******* Perlite microspheres, average particle sizes between 50-100 µm | | | | | | | |

The dispersions of polymers and particulates in organic solvent were filled into aerosol cans and closed before adding the propellant. Separately, the compositions were filled into a viscometer as disclosed in EP2746745 and the viscosities were measured. They were to be in the range of 4,000-5,000 mPas.

Human hair streaks (Caucasian, 21 cm long, 2 g per bundle) were purchased from Fischbach + Miller Haar, Laupheim, Germany. The hair streaks were washed with a commercially available shampoo under the brand name Goldwell Dualsenses Ultra Volume Shampoo and then blow-dried. Onto the dry hair streaks, the compositions from above were sprayed until each hair streak received 0.5 g of solids (particulates, polymers). The hair streaks were then allowed to air-dry.

Surface roughness of hair fibers was analyzed with a Zwick Roell device. A hair streak was pulled through a defined hole. The force that was needed to pull the hair streak through the analysis cell was measured [mN]. The friction is understood as surface roughness. Roughness correlates with volume and body as a rough texture correlates with the amount of volume increase. Each hair streak was measured for 10 times and averages are presented below:

| **Parameter** | **Inventive comp. 1** | **Inventive comp. 2** | **Inventive comp. 3** | **Inventive comp. 4** | **Inventive comp. 5** | **Comparative comp. 1** |
|---|---|---|---|---|---|---|
| | **Force [mN]** | | | | | |
| **Average** | 76.7 | 68.4 | 52.4 | 71.0 | 51.1 | 18.8 |
| **SD** | 6.1 | 6.1 | 5.1 | 3.7 | 4.2 | 1.4 |

As revealed by the texture measurements, the inventive compositions had at least twice the roughness in comparison to the comparative composition. Thus, the inventive compositions conferred to hair higher volume and body.

### Example 2

The following compositions were prepared by conventional formulation and mixing techniques:

| **Ingredients** | | **Inventive comp. 6** | **Comp. comp. 2** | **Comp. comp. 3** |
|---|---|---|---|---|
| | | **% by weight** | | |
| **Non-ionic film forming polymer** | **VP/VA copolymer** | 1.0 | 1.0 | 1.0 |
| **Anionic film forming polymer** | **Acrylates/t-Butylacrylamide copolymer*** | 1.5 | 1.5 | 1.5 |
| **Particulates** | **Amorphous silica** | 1.5 | - | 1.5 |
| **Solvent** | **Ethanol** | 15.0 | 15.0 | 7.5 |
| | **Water** | - | - | 7.5 |
| **Propellant** | **Dimethylether** | Ad 100.0 | | |

| | | | | |
|---|---|---|---|---|
| * Luviskol VA 64 ** Ultrahold Power by BASF Corp. | | | | |

The dispersions of polymers and particulates (if present) in organic solvent were filled into aerosol cans. The cans were closed before adding the propellant.

The compositions were sprayed onto a half-side of mannequin hair wherein the treated side received 0.5 g solids in total. The mannequins had identical amount of hair. The hair was then allowed to air-dry and drying time was recorded. The mannequin heads were photographed and the images were analyzed with Imagej software. Hair volume of the treated sides and untreated sides were measured in pixels. Then the ratio of treated to untreated side was calculated to yield a hair volume factor. The results were as follows:

| **Composition** | **Drying time [s]** | **Hair volume factor** |
|---|---|---|
| **Inventive comp. 6** | < 60 s | 1.73 |
| **Comp. comp. 2** | < 60 s | 1.30 |
| **Comp. comp. 3** | > 120 s | 1.35 |

As illustrated by the examples above, the inventive composition is quick-drying and enhances hair volume much better in comparison to the comparative compositions.

The following examples are within the scope of the present invention.

### Example 3

| | **% by weight** |
|---|---|
| VPNA copolymer* | 0.5 |
| Acrylates/t-butylacrylamide | 0.5 |
| Copolymer** | |
| Octylacrylamide/Acrylates/ | 0.5 |
| Butylaminoethyl Methacrylate | |
| Copolymer*** | |
| Amorphous silica | 0.5 |
| HC Red 18 | 0.05 |
| Ethanol | 10.0 |
| Carbon dioxide | ad 100.0 |

| | |
|---|---|
| * Luviskol VA 64 ** Ultrahold Power by BASF Corp. *** Amphomer by AkzoNobel Corp. | |

### Example 4

| | **% by weight** |
|---|---|
| PVP 90* | 1.0 |
| Acrylates/t-butylacrylamide Copolymer** | 0.2 |
| Amorphous silica | 2.0 |
| Ethanol | 20.0 |
| Dimethylether: isobutane (50:50) | ad 100.0 |

| | |
|---|---|
| *Luviskol K90 by BASF Corp. **Ultrahold Power by BASF Corp. | |

### Example 5

| | **% by weight** |
|---|---|
| VPNA copolymer* | 4.0 |
| Polysilicone 28 | 0.2 |
| Acrylates/t-butylacrylamide Copolymer** | 0.8 |
| Amorphous silica | 3.0 |
| Ethanol | 45.0 |
| Dimethylether | ad 100.0 |

| | |
|---|---|
| * Luviskol VA 64 ** Ultrahold Power by BASF Corp. | |

### Example 6

| | **% by weight** |
|---|---|
| VPNA copolymer* | 1.0 |
| Acrylates/t-butylacrylamide Copolymer** | 4.0 |
| Amorphous silica | 3.0 |
| Ethanol | 45.0 |
| Dimethylether | ad 100.0 |

| | |
|---|---|
| * Luviskol VA 64 ** Ultrahold Power by BASF Corp. | |

## Claims

1. An aerosol composition comprising:
• One or more non-silicone non-ionic film forming polymer(s),
• One or more anionic film forming polymer(s), and/or its/their salt(s), and/or their mixtures,
• One or more organic and/or inorganic particulate being insoluble in organic solvents having a boiling point in the range of 50°C to 120°C under atmospheric conditions,
• One or more organic solvent(s) having a boiling point in the range of 50°C to 120°C under atmospheric conditions,
• One or more propellant(s),
wherein the composition comprises less than 5% by weight of water, preferably less than 1% by weight of water, calculated to the total of the composition, more preferably it is anhydrous.

2. The composition according to claim 1 **characterized in that** non-ionic film forming polymers comprise monomer units selected from vinylpyrrolidone, vinylacetate, and vinylcaprolactam, preferably it is vinylacetate / vinylpyrrolidone copolymer.

3. The composition according to claims 1 and/or 2 **characterized in that** the total concentration of one or more non-silicone non-ionic film forming polymer(s) is in the range of 0.1% to 10% by weight, preferably in the range of 0.2% to 8% by weight, more preferably in the range of 0.5% to 5% by weight, calculated to the total of the composition.

4. The composition according to any of the preceding claims **characterized in that** the composition further comprises a cationic polymer, preferably a cationic silicone polymer, more preferably Polysilicone 9 and/or Polysilicone 28.

5. The composition according to any of the preceding claims **characterized in that** the one or more anionic film forming polymer(s) have acrylate and/or alkyl acrylate and/or acrylamide and/or alkyl acrylamide monomers and/or their salts, the anionic film forming polymer(s) preferably being acrylates/t-butylacrylamide copolymer, and/or its salts, and/or its mixtures.

6. The composition according to any of the preceding claims **characterized in that** the total concentration of anionic film forming polymers is in the range of 0.1% to 10% by weight, preferably in the range of 0.2% to 8% by weight, more preferably in the range of 0.5% to 5% by weight, calculated to the total of the composition.

7. The composition according to any of the preceding claims **characterized in that** the one or more organic and/or inorganic particulate has an average particle size in the range of 1 µm to 250 µm, preferably in the range of 10 µm to 100 µm.

8. The composition according to any of the preceding claims **characterized in that** the one or more organic and/or inorganic particulate does not confer color to hair, judged by the naked human eye under daylight conditions while spraying 0.5 g of the composition onto 2 g of keratin fibers.

9. The composition according to any of the preceding claims **characterized in that** the total concentration of one or more organic and/or inorganic particulate is in the range of 0.25% to 10% by weight, preferably in the range of 0.5% to 8% by weight, more preferably in the range of 1% to 5% by weight, calculated to the total of the composition.

10. The composition according to any of the preceding claims **characterized in that** the inorganic particulate is amorphous silica and/or calcium carbonate.

11. The composition according to any of the preceding claims **characterized in that** the organic particulate is a vegetable starch and/or its synthetically modified derivatives.

12. The composition according to any of the preceding claims **characterized in that** the one or more solvent(s) are ethanol, n-propanol, iso-propanol, and/or their mixtures.

13. The composition according to any of the preceding claims **characterized in that** the total concentration of propellants is more than 50% by weight, preferably in the range of 60% to 95% by weight, more preferably in the range of 70% to 90% by weight, calculated to the total of the composition.

14. A method for styling and/or increasing volume and/or body of keratin fibers, preferably human keratin fibers, more preferably human hair, **characterized in that** it comprises the steps of:
a) optionally shampooing and drying the keratin fibers,
b) applying to dry keratin fibers a composition according to any of the claims 1 to 13,
c) optionally styling the keratin fibers using a comb or brush.

15. A vaporizer comprising the composition according to any of the claims 1 to 13.

## Patentansprüche

1. Aerosolzusammensetzung, umfassend:
- ein oder mehrere nicht-ionische(s) filmbildende(s) Nicht-Silikon-Polymer(e),
- ein oder mehrere anionische(s) filmbildende(s) Polymer(e) und/oder dessen/deren Salz(e) und/oder deren Mischungen,
- einen oder mehrere organische und/oder anorganische Partikel, die in organischen Lösungsmitteln mit einem Siedepunkt im Bereich von 50°C bis 120°C unter atmosphärischen Bedingungen unlöslich sind,
- ein oder mehrere organische(s) Lösungsmittel mit einem Siedepunkt im Bereich von 50°C bis 120°C unter atmosphärischen Bedingungen,
- ein oder mehrere Treibmittel,
wobei die Zusammensetzung weniger als 5 Gew.-% Wasser, bevorzugt weniger als 1 Gew.-% Wasser, berechnet auf die Gesamtmenge der Zusammensetzung, umfasst und mehr bevorzugt wasserfrei ist.

2. Zusammensetzung gemäß Anspruch 1, **dadurch gekennzeichnet, dass** die nicht-ionischen filmbildenden Polymere Monomereinheiten umfassen, die aus Vinylpyrrolidon, Vinylacetat und Vinylcaprolactam ausgewählt sind, bevorzugt ist es ein Vinylacetat/Vinylpyrrolidon-Copolymer.

3. Zusammensetzung gemäß Anspruch 1 und/oder 2, **dadurch gekennzeichnet, dass** die Gesamtkonzentration des einen oder der mehreren nicht-ionischen filmbildenden Nicht-Silikon-Polymer(s/e) im Bereich von 0,1 Gew.-% bis 10 Gew.-%, bevorzugt im Bereich von 0,2 Gew.-% bis 8 Gew.-%, mehr bevorzugt im Bereich von 0,5 Gew.-% bis 5 Gew.-%, berechnet auf die Gesamtmenge der Zusammensetzung, liegt.

4. Zusammensetzung gemäß einem der vorhergehenden Ansprüche, **dadurch gekennzeichnet, dass** die Zusammensetzung weiterhin ein kationisches Polymer, bevorzugt ein kationisches Silikon-Polymer, mehr bevorzugt Polysilikon 9 und/oder Polysilikon 28, umfasst.

5. Zusammensetzung gemäß einem der vorhergehenden Ansprüche, **dadurch gekennzeichnet, dass** das eine oder die mehreren anionische(n) filmbildende(n) Polymer(e) Acrylat- und/oder Alkylacrylat- und/oder Acrylamid- und/oder Alkylacrylamid-Monomere und/oder deren Salze aufweist/aufweisen, wobei das/die anionische(n) filmbildende(n) Polymer(e) bevorzugt Acrylat/t-Butylacrylamid-Copolymer und/oder dessen Salze und/oder dessen Mischungen ist/sind.

6. Zusammensetzung gemäß einem der vorhergehenden Ansprüche, **dadurch gekennzeichnet, dass** die Gesamtkonzentration der anionischen filmbildenden Polymere im Bereich von 0,1 bis 10 Gew.-%, bevorzugt im Bereich von 0,2 bis 8 Gew.-%, mehr bevorzugt im Bereich von 0,5 bis 5 Gew.-%, berechnet auf die Gesamtmenge der Zusammensetzung, liegt.

7. Zusammensetzung gemäß einem der vorangehenden Ansprüche, **dadurch gekennzeichnet, dass** der eine oder die mehreren organischen und/oder anorganischen Partikel eine durchschnittliche Partikelgröße im Bereich von 1 µm bis 250 µm, bevorzugt im Bereich von 10 µm bis 100 µm, aufweisen.

8. Zusammensetzung gemäß einem der vorhergehenden Ansprüche, **dadurch gekennzeichnet, dass** der eine oder die mehreren organischen und/oder anorganischen Partikel dem Haar keine Farbe verleihen, beurteilt mit dem bloßen menschlichen Auge unter Tageslichtbedingungen beim Aufsprühen von 0,5 g der Zusammensetzung auf 2 g Keratinfasern.

9. Zusammensetzung gemäß einem der vorhergehenden Ansprüche, **dadurch gekennzeichnet, dass** die Gesamtkonzentration des einen oder der mehreren organischen und/oder anorganischen Partikel im Bereich von 0,25 bis 10 Gew.-%, bevorzugt im Bereich von 0,5 bis 8 Gew.-%, mehr bevorzugt im Bereich von 1 bis 5 Gew.-%, berechnet auf die Gesamtmenge der Zusammensetzung, liegt.

10. Zusammensetzung gemäß einem der vorhergehenden Ansprüche, **dadurch gekennzeichnet, dass** die anorganischen Partikel amorphes Siliciumoxid und/oder Calciumcarbonat sind.

11. Zusammensetzung gemäß einem der vorangehenden Ansprüche, **dadurch gekennzeichnet, dass** die organischen Partikel eine pflanzliche Stärke und/oder deren synthetisch modifizierte Derivate sind.

12. Zusammensetzung gemäß einem der vorhergehenden Ansprüche, **dadurch gekennzeichnet, dass** das eine oder die mehreren Lösungsmittel Ethanol, n-Propanol, Isopropanol und/oder deren Mischungen ist/sind.

13. Zusammensetzung gemäß einem der vorhergehenden Ansprüche, **dadurch gekennzeichnet, dass** die Gesamtkonzentration der Treibmittel mehr als 50 Gew.-%, bevorzugt im Bereich von 60 bis 95 Gew.-%, mehr bevorzugt im Bereich von 70 bis 90 Gew.-%, berechnet auf die Gesamtmenge der Zusammensetzung, beträgt.

14. Verfahren zum Stylen und/oder zur Erhöhung des Volumens und/oder der Fülle von Keratinfasern, bevorzugt menschlichen Keratinfasern, mehr bevorzugt menschlichem Haar, **dadurch gekennzeichnet, dass** es die Schritte umfasst:
a) optional das Shampoonieren und das Trocknen der Keratinfasern,
b) das Auftragen einer Zusammensetzung gemäß einem der Ansprüche 1 bis 13 auf trockene Keratinfasern,
c) optional das Stylen der Keratinfasern unter Verwendung eines Kamms oder einer Bürste.

15. Verdampfer, umfassend die Zusammensetzung gemäß einem der Ansprüche 1 bis 13.

## Revendications

1. Composition d'aérosol comprenant :
• un ou plusieurs polymère(s) filmogène(s) non ionique(s) sans silicone,
• un ou plusieurs polymère(s) filmogène(s) anionique(s), et/ou son(ses)/leur(leurs) sel(s), et/ou leurs mélanges,
• une ou plusieurs particule organique et/ou inorganique insoluble dans des solvants organiques présentant un point d'ébullition dans la plage de 50 °C à 120 °C dans des conditions atmosphériques,
• un ou plusieurs solvant(s) organique(s) présentant un point d'ébullition dans la plage de 50 °C à 120 °C dans des conditions atmosphériques,
• un ou plusieurs propulseur(s),
dans laquelle la composition comprend moins de 5 % en poids d'eau, de préférence moins de 1 % en poids d'eau, calculé par rapport au total de la composition, plus préférablement elle est anhydre.

2. Composition selon la revendication 1 **caractérisée en ce que** les polymères filmogènes non ioniques comprennent des unités monomères sélectionnées parmi la pyrrolidone de vinyle, l'acétate de vinyle et le caprolactame de vinyle, de préférence il s'agit d'un copolymère d'acétate de vinyle/pyrrolidone de vinyle.

3. Composition selon les revendications 1 et/ou 2 **caractérisée en ce que** la concentration totale d'un ou plusieurs polymère(s) filmogène(s) non ionique(s) sans silicone se situe dans la plage de 0,1 % à 10 % en poids, de préférence dans la plage de 0,2 % à 8 % en poids, plus préférablement dans la plage de 0,5 % à 5 % en poids, calculée par rapport au total de la composition.

4. Composition selon l'une quelconque des revendications précédentes **caractérisée en ce que** la composition comprend en outre un polymère cationique, de préférence un polymère de silicone cationique, plus préférablement du Polysilicone 9 et/ou du Polysilicone 28.

5. Composition selon l'une quelconque des revendications précédentes **caractérisée en ce que** le(s) un ou plusieurs polymère(s) filmogène(s) anionique(s) présentent des monomères d'acrylate et/ou d'acrylate d'alkyle et/ou d'acrylamide et/ou d'acrylamide d'alkyle et/ou leurs sels, le(les) polymère(s) filmogène(s) anionique(s) étant de préférence un copolymère d'acrylates/t-butylacrylamide, et/ou ses sels, et/ou ses mélanges.

6. Composition selon l'une quelconque des revendications précédentes **caractérisée en ce que** la concentration totale de polymères filmogènes anioniques se situe dans la plage de 0,1 % à 10 % en poids, de préférence dans la plage de 0,2 % à 8 % en poids, plus préférablement dans la plage de 0,5 % à 5 % en poids, calculée par rapport au total de la composition.

7. Composition selon l'une quelconque des revendications précédentes **caractérisée en ce que** la/les une ou plusieurs particule organique et/ou inorganique présente une taille de particule moyenne dans la plage de 1 µm à 250 µm, de préférence dans la plage de 10 µm à 100 µm.

8. Composition selon l'une quelconque des revendications précédentes **caractérisée en ce que** la/les une ou plusieurs particule organique et/ou inorganique ne confère pas de couleur aux cheveux, jugées à l'œil nu humain à la lumière du jour pendant la pulvérisation de 0,5 g de la composition sur 2 g de fibres de kératine.

9. Composition selon l'une quelconque des revendications précédentes **caractérisée en ce que** la concentration totale d'une ou plusieurs particule organique et/ou inorganique se situe dans la plage de 0,25 % à 10 % en poids, de préférence dans la plage de 0,5 % à 8 % en poids, plus préférablement dans la plage de 1 % à 5 % en poids, calculée par rapport au total de la composition.

10. Composition selon l'une quelconque des revendications précédentes **caractérisée en ce que** la particule inorganique est de la silice amorphe et/ou du carbonate de calcium.

11. Composition selon l'une quelconque des revendications précédentes **caractérisée en ce que** la particule organique est de l'amidon végétal et/ou ses dérivés synthétiquement modifiés.

12. Composition selon l'une quelconque des revendications précédentes **caractérisée en ce que** le(s) un ou plusieurs solvant(s) sont de l'éthanol, du n-propanol, de l'iso-propanol et/ou leurs mélanges.

13. Composition selon l'une quelconque des revendications précédentes **caractérisée en ce que** la concentration totale de propulseurs est de plus de 50 % en poids, de préférence dans la plage de 60 % à 95 % en poids, plus préférablement dans la plage de 70 % à 90 % en poids, calculée par rapport au total de la composition.

14. Procédé destiné à coiffer et/ou augmenter le volume et/ou le corps de fibres de kératine, de préférence des fibres de kératine humaines, plus préférablement des cheveux humains, **caractérisé en ce qu'**il comprend les étapes consistant à :
a) facultativement shampouiner et faire sécher les fibres de kératine,
b) appliquer aux fibres de kératine sèches une composition selon l'une quelconque des revendications 1 à 13,
c) facultativement coiffer les fibres de kératine à l'aide d'un peigne ou d'une brosse.

15. Vaporisateur comprenant la composition selon l'une quelconque des revendications 1 à 13.
